(19) Europäisches Patentamt
European Patent Office
Office européen des brevets

(11) Veröffentlichungsnummer: **0 581 132 A2**

# (12) EUROPÄISCHE PATENTANMELDUNG

(21) Anmeldenummer: **93111386.4**

(22) Anmeldetag: **15.07.93**

(51) Int. Cl.$^5$: **C07D 239/60**, C07D 401/12, C07D 251/30, A01N 43/54

(30) Priorität: **28.07.92 DE 4224928**

(43) Veröffentlichungstag der Anmeldung: **02.02.94 Patentblatt 94/05**

(84) Benannte Vertragsstaaten: **BE CH DE FR GB IT LI NL**

(71) Anmelder: **BAYER AG**

**D-51368 Leverkusen(DE)**

(72) Erfinder: **Drewes, Mark Wilhelm, Dr.**
**Talstrasse 54**
**D-40764 Langenfeld(DE)**
Erfinder: **Kirsten, Rolf, Dr.**
**Carl-Langhans-Strasse 27**
**d-40789 Monheim(DE)**
Erfinder: **Santel, Hans-Joachim, Dr.**
**Grünstrasse 9a**
**D-51371 Leverkusen(DE)**
Erfinder: **Lürssen, Klaus, Dr.**
**August-Kierspel-Strasse 145**
**D-51469 Bergisch Gladbach(DE)**
Erfinder: **Schmidt, Robert R., Dr.**
**Im Waldwinkel 110**
**D-51467 Bergisch Gladbach(DE)**

## (54) Substituierte Bicyclo[3.1.0]hexane und ihre Verwendung als Herbizide.

(57) Die Erfindung betrifft neuartige substituierte Bicyclo[3.1.0]hexane der Formel

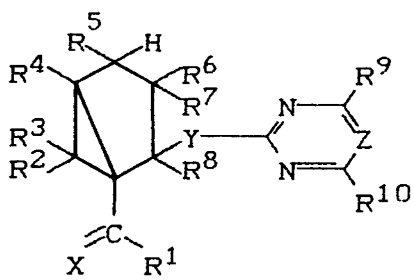

(I)

in welcher

| | |
|---|---|
| $R^1$ | z.B. für Hydroxy oder Alkoxy steht, |
| $R^2$ bis $R^8$ | z.B. für Wasserstoff oder Alkyl stehen, |
| $R^9$ und $R^{10}$ | z.B. für Methyl oder Methoxy stehen, |
| X | z.B. für Sauerstoff steht, |
| Y | z.B. für Sauerstoff oder Schwefel steht und |
| Z | z.B. für CH oder N steht, |

sowie Salze der freien Säure ($R^1$ = OH) und deren weitere funktionelle Derivate, ferner Verfahren und neue Zwischenprodukte zu ihrer Herstellung und ihre Verwendung als Herbizide.

Außerdem wird ein neuartiges Verfahren zur Herstellung von bestimmten, als Zwischenprodukte benötigten 2-Ketobicyclo[3.1.0]hexanen beschrieben, ausgehend von entsprechend substituierten $\alpha,\beta$-ungesättigten Carbonylverbindungen (vom Typ des Methacroleins) und $\beta$-Ketosäureestern (vom Typ der Acetessigsäureester).

EP 0 581 132 A2

Die Erfindung betrifft neuartige substituierte Bicyclo[3.1.0]hexane, ein Verfahren und neue Zwischenprodukte zu ihrer Herstellung und ihre Verwendung als Herbizide.

Bicyclo[3.1.0]hexanderivate haben bisher keine Bedeutung als Herbizide erlangt.

Es wurden nun neuartige substituierte Bicyclo[3.1.0]hexane der allgemeinen Formel (I) gefunden,

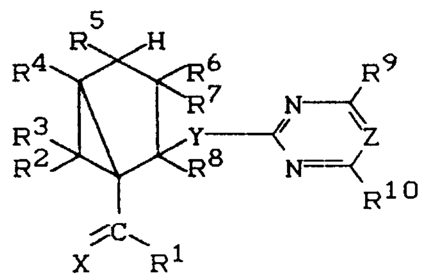

(I)

in welcher

- $R^1$ für Wasserstoff, Hydroxy, Amino oder für einen jeweils gegebenenfalls substituierten Rest aus der Reihe Alkyl, Alkoxy, Alkenyloxy, Alkinyloxy, Aryloxy, Heteroaryloxy, Alkylthio, Arylthio, Heteroarylthio, Alkylamino, Dialkylamino, Alkylsulfonylamino, Arylsulfonylamino oder Hydrazino steht,
- $R^2$ für Wasserstoff, Halogen, Alkyl, Alkoxy, Aryl oder Alkoxycarbonyl steht,
- $R^3$ für Wasserstoff oder Alkyl oder - wenn $R^2$ Wasserstoff, Alkyl oder Halogen bedeutet - auch für Halogen steht,
- $R^4$ für Wasserstoff, Halogen, Alkyl, Aryl oder Alkoxycarbonylalkyl steht,
- $R^5$ für Wasserstoff, Alkyl oder Aryl steht,
- $R^6$ für Wasserstoff, Hydroxy, Amino, Halogen, Cyano, Alkyl, Alkoxy, Aryl oder Alkoxycarbonyl steht,
- $R^7$ für Wasserstoff oder Alkyl oder - wenn $R^6$ Wasserstoff, Alkyl oder Halogen bedeutet - auch für Halogen steht,
- $R^8$ für Wasserstoff, Cyano, Alkyl, Aryl oder Alkoxycarbonylalkyl steht,
- $R^9$ und $R^{10}$ gleich oder verschieden sind und für Wasserstoff, Halogen oder für einen jeweils gegebenenfalls substituierten Rest der Reihe Alkyl, Alkoxy, Alkylthio, Alkylamino, Dialkylamino, Aryl oder Aryloxy stehen,
- X für Sauerstoff oder Schwefel oder - wenn $R^1$ Wasserstoff oder Alkyl bedeutet - auch für eine der nachstehenden Gruppierungen steht:

  N-$R^{11}$ oder

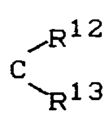

,

wobei

- $R^{11}$ für Wasserstoff, Hydroxy, Amino oder für einen jeweils gegebenenfalls substituierten Rest der Reihe Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Aralkylamino, N-Alkyl-N-arylamino, Hetarylamino, Hetarylcarbonylamino, Arylcarbonylamino oder Arylsulfonylamino steht,
- $R^{12}$ für Wasserstoff, Halogen, Cyano, Carboxyl, Alkoxycarbonyl, Alkylcarbonylamino oder Dialkoxyphosphoryl steht und
- $R^{13}$ für Formyl, Cyano, Carboxyl, Hydroxymethyl, Carbamoyl oder für einen jeweils gegebenenfalls substituierten Rest der Reihe Alkoxycarbonyl, Cycloalkyloxycarbonyl, Alkylthiocarbonyl, Alkylaminocarbonyl, Cycloalkylaminocarbonyl, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl,

Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl, Phthalimidoxycarbonyl oder $R^{12}$ zusammen mit $R^{13}$ für die Gruppierung $-CO-O-(CH_2)_n-$ steht, wobei n für die Zahlen 1 bis 4 steht,

Y für Sauerstoff, Schwefel, Imino (NH) oder Alkylimino (N-Alkyl) steht, und

Z für Stickstoff oder die Gruppierung $C-R^{14}$ steht, worin $R^{14}$ für Wasserstoff, Halogen, Alkyl oder Alkoxy steht,

sowie Salze der freien Sauren ($R^1$ = OH) und die Anhydride der freien Säuren.

Die Verbindungen der Formel (I) können in unterschiedlichen isomeren Formen (d.h. in verschiedenen geometrischen und optischen Isomerenformen) existieren; alle diese Isomeren, welche der generellen Strukturformel (I) entsprechen, sind Gegenstand der Erfindung.

Es wurde weiter gefunden, daß man die neuen Bicyclo[3.1.0]hexane der allgemeinen Formel (I) erhält, wenn man Bicyclo[3.1.0]-hexane der Formel (II),

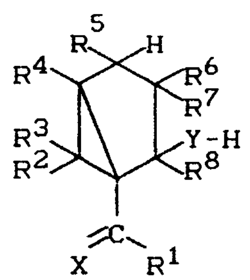

(II)

in welcher

$R^1$, $R^2$, $R^3$, $R^4$, $R^5$, $R^6$, $R^7$, $R^8$, X und Y die oben bei Formel (I) angegebenen Bedeutungen haben,

mit Azinen der Formel (III),

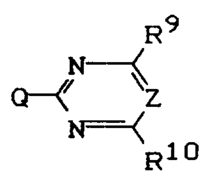

(III)

in welcher

$R^9$, $R^{10}$ und Z die oben bei Formel (I) angegebenen Bedeutungen haben und

Q für eine nucleofuge Abgangsgruppe steht,

gegebenenfalls in Gegenwart eines Säureakzeptors und gegebenenfalls in Gegenwart eines Verdünnungsmittels umsetzt und die freien Säuren ($R^1$ = OH) gegebenenfalls - nach üblichen Methoden - in ihre Salze oder andere funktionelle Derivate überführt.

Die neuen substituierten Bicyclo[3.1.0]hexane der Formel (I) zeichnen sich durch starke herbizide Wirksamkeit aus.

Die Erfindung betrifft vorzugsweise Verbindungen der Formel (I), in welcher

$R^1$ für Wasserstoff, Hydroxy, Amino oder für einen jeweils gegebenenfalls durch Halogen oder $C_1$-$C_5$-Alkoxy substituierten Rest aus der Reihe $C_1$-$C_5$-Alkyl, $C_1$-$C_5$-Alkoxy, $C_3$-$C_5$-Alkenyloxy, $C_3$-$C_5$-Alkinyloxy, Cyano-$C_1$-$C_3$-alkoxy, Methylthio-$C_1$-$C_3$-alkoxy, Phenyloxy, Pyridyloxy, (Nitro)-Chinolyloxy, Isochinolyloxy, $C_1$-$C_5$-Alkylthio, Phenylthio, Pyrimidinylthio, $C_1$-$C_5$-Alkylamino, ($C_1$-$C_2$-Alkoxycarbonyl)-$C_1$-$C_5$-alkylamino, Phenyl-($C_1$-$C_5$-alkylamino), Benzylamino, Phenethylamino, Di-($C_1$-$C_4$-alkyl)-amino, ($C_1$-$C_5$-Alkyl)-sulfonylamino, ($c_6$-$C_{10}$-Aryl)-sulfonylamino oder für einen gegebenenfalls durch $C_1$-$C_2$-Alkyl substituierten Hydrazinorest steht,

$R^2$ für Wasserstoff, Halogen, $C_1$-$C_5$-Alkyl, $C_1$-$C_5$-Alkoxy, Phenyl oder ($C_1$-$C_5$-Alkoxy)-carbonyl steht,

$R^3$ für Wasserstoff oder $C_1$-$C_5$-Alkyl oder - wenn $R^2$ Wasserstoff, Alkyl oder Halogen bedeutet - auch für Halogen steht,

$R^4$ für Wasserstoff, Halogen, $C_1$-$C_5$-Alkyl oder Phenyl steht,

$R^5$ für Wasserstoff, $C_1$-$C_5$-Alkyl oder Phenyl steht,

$R^6$ für Wasserstoff, Hydroxy, Amino, Halogen, Cyano, $C_1$-$C_5$-Alkyl, $C_1$-$C_5$-Alkoxy, Phenyl oder ($C_1$-$C_5$-Alkoxy)-carbonyl steht,

$R^7$ für Wasserstoff oder $C_1$-$C_5$-Alkyl oder - wenn $R^6$ Wasserstoff, Alkyl oder Halogen bedeutet - auch für Halogen steht,

$R^8$ für Wasserstoff, Cyano, $C_1$-$C_5$-Alkyl, Phenyl oder ($C_2$-$C_6$-Alkoxycarbonyl)-methyl steht,

$R^9$ und $R^{10}$ gleich oder verschieden sind und für Wasserstoff, Halogen oder für einen jeweils gegebenenfalls durch Halogen oder $C_1$-$C_3$-Alkoxy substituierten Rest der Reihe $C_1$-$C_4$-Alkyl, $C_1$-$C_4$-Alkoxy, $C_1$-$C_4$-Alkylthio, $C_1$-$C_4$-Alkylamino, Di-($C_1$-$C_4$-alkyl)-amino, Phenyl oder Phenoxy stehen,

X für Sauerstoff oder Schwefel oder - wenn $R^1$ Wasserstoff oder Alkyl bedeutet - auch für eine der nachstehenden Gruppierungen steht:

N-$R^{11}$ oder

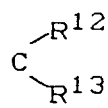

wobei

$R^{11}$ für Wasserstoff, Hydroxy, Amino oder für einen jeweils gegebenenfalls durch Halogen substituierten Rest der Reihe $C_1$-$C_6$-Alkyl, $C_3$-$C_6$ Alkenyl, $C_3$-$C_6$-Alkinyl, $C_1$-$C_6$-Alkoxy, $C_3$-$C_6$-Alkenyloxy, $C_1$-$C_4$-Alkoxy-carbonyl-$C_1$-$C_2$-alkoxy, $C_1$-$C_6$-Alkylamino, Di-($C_1$-$C_2$-alkyl)-amino, $C_1$-$C_6$-Alkylcarbonylamino, $C_1$-$C_6$-Alkoxycarbonylamino, $C_1$-$C_6$-Alkylsulfonylamino, oder für jeweils gegebenenfalls durch Nitro, Amino, Cyano, Carboxyl, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituierten Rest der Reihe Phenyl, Phenyl-$C_1$-$C_4$-alkyl, Phenoxy, Phenyl-$C_1$-$C_4$-alkoxy, Phenylamino, Phenyl-$C_1$-$C_4$-alkylamino, N-$C_1$-$C_4$-alkyl)-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^{12}$ für Wasserstoff, Halogen, Cyano, Carboxyl, $C_1$-$C_6$-Alkoxycarbonyl, $C_1$-$C_6$-Alkylcarbonylamino oder Di-($C_1$-$C_4$-alkoxy)-phosphoryl steht und

$R^{13}$ für Formyl, Cyano, Carboxyl, Hydroxymethyl, Carbamoyl oder für einen jeweils gegebenenfalls durch Halogen, Carboxyl oder $C_1$-$C_4$-Alkoxycarbonyl substituierten Rest der Reihe $C_1$-$C_6$-Alkoxycarbonyl, $C_5$-$C_6$-Cycloalkyloxycarbonyl, $C_1$-$C_6$-Alkylthio-carbonyl, $C_1$-$C_6$-Alkylaminocarbonyl, $C_5$-$C_6$-Cycloalkylaminocarbonyl, Di-($C_1$-$C_2$-alkyl)-aminocarbonyl, $C_1$-$C_4$-Alkylaminocarbonyl-$C_1$-$C_4$-alkoxycarbonyl, Di-($C_1$-$C_2$-alkyl )-aminocarbonyl-$C_1$-$C_4$-alkoxycarbonyl, Phenylaminocarbonyl-$C_1$-$C_4$-alkoxycarbonyl, N-Methyl-N-Phenylaminocarbonyl-$C_1$-$C_4$-alkoxycarbonyl, oder für einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl,
oder für einen jeweils gegebenenfalls durch Nitro, Amino, Cyano, Carboxyl, Halogen, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkoxy-carbonyl und/oder Di-($C_1$-$C_2$-alkyl)-amino substituierten Rest der Reihe Phenoxycarbonyl, Phenyl-$C_1$-$C_4$-alkoxycarbonyl, Furylmethoxycarbonyl, Thienylmethoxycarbonyl, Phenylthiocarbonyl, Phenyl-$C_1$-$C_4$-alkylthio-carbonyl, Phenylaminocarbonyl, Phenyl-$C_1$-$C_4$-alkylaminocarbonyl,N-($C_1$-$C_4$-Alkyl)-N-phenylaminocarbonyl oder Phenylhydrazinocarbonyl, $C_1$-$C_4$-Alkylhydrazinocarbonyl, für Phthalimidoxycarbonyl oder $R^{12}$ zusammen mit $R^{13}$ für die Gruppierung $-CO-O(CH_2)_n-$ steht, wobei

n für die Zahlen 1 bis 4 steht,

Y für Sauerstoff, Schwefel, Imino (NH) oder Methylimino ($NCH_3$) steht und

Z für Stickstoff oder die Gruppierung C-$R^{14}$ steht,

worin

$R^{14}$ für Wasserstoff, Fluor, Chlor, Methyl oder Methoxy steht.

Gegenstand der Erfindung sind weiter vorzugsweise Natrium-, Kalium-, Magnesium-, Calcium-, Ammonium-, $C_1$-$C_4$-Alkyl-ammonium, Di-($C_1$-$C_4$-alkyl)-ammonium-, Tri($C_1$-$C_4$-alkyl)-ammonium-, $C_5$- oder $C_6$-Cy-

cloalkylammonium- und Di-($C_1$-$C_2$-alkyl)-benzylammonium-Salze von Verbindungen der Formel (I), in welcher

$R^1$ für OH steht und $R^2$ bis $R^{14}$, X, Y und Z die obengenannten bevorzugten Bedeutungen haben.

Besonders bevorzugte Verbindungen gemäß der Erfindung sind solche substituierten Bicyclo[3.1.0]-hexane der obigen Formel (I), worin

$R^1$ für Wasserstoff, Hydroxy, Amino oder für einen jeweils gegebenenfalls durch Fluor, Chlor, Methoxy oder Ethoxy substituierten Rest aus der Reihe Methyl, Ethyl, Propyl, Isopropyl, Methoxy, Ethoxy, Propoxy, Isopropoxy, Cyanomethoxy, Allyloxy, Propargyloxy, Methylthio-ethoxy, (Nitro)-Chinolyloxy, (Fluor)Phenylthio, (Dimethoxy)-Pyrimidinylthio, Methylamino, Ethylamino, Propylamino, Isopropylamino, Dimethylamino, Diethylamino, Methoxycarbonylmethylamino, Methoxycarbonylethylamino, (Chlor)-Phenylethylamino, Benzylamino, Methylsulfonylamino, Ethylsulfonylamino, Phenylsulfonylamino oder (Dimethyl)-Hydrazino steht,

$R^2$ für Wasserstoff, Fluor, Chlor, Brom, Methyl, Ethyl, Propyl, Isopropyl, Phenyl, Methoxycarbonyl oder Ethoxycarbonyl steht,

$R^3$ für Wasserstoff, Methyl, Ethyl, Propyl oder - wenn $R^2$ Wasserstoff, Alkyl oder Halogen bedeutet - auch für Fluor, Chlor oder Brom steht,

$R^4$ für Wasserstoff, Fluor, Chlor, Brom, Methyl, Ethyl, Propyl, Isopropyl oder Phenyl steht,

$R^5$ für Wasserstoff, Methyl, Ethyl, Propyl, Isopropyl oder Phenyl steht,

$R^6$ für Wasserstoff, Chlor, Fluor, Methyl, Ethyl, Methoxy, Ethoxy, Phenyl, Methoxycarbonyl oder Ethoxycarbonyl steht,

$R^7$ für Wasserstoff, Methyl oder Ethyl oder - wenn $R^6$ Wasserstoff, Alkyl oder Halogen bedeutet - auch für Chlor oder Fluor steht,

$R^8$ für Wasserstoff, Cyano, Methyl, Ethyl, Phenyl, Methoxycarbonylmethyl oder Ethoxycarbonylmethyl steht,

$R^9$ und $R^{10}$ gleich oder verschieden sind und für Wasserstoff, Chlor, Methyl, Ethyl, Trifluormethyl, Methoxymethyl, Methoxy, Ethoxy, Difluormethoxy, Methylthio, Methylamino, Ethylamino, Dimethylamino, Phenyl oder Phenoxy stehen,

X für Sauerstoff oder Schwefel oder - wenn $R^1$ Wasserstoff oder Alkyl bedeutet - auch für eine der nachstehenden Gruppierungen steht

N-$R^{11}$ oder

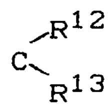

wobei

$R^{11}$ für Wasserstoff, Hydroxy, Amino, für Methyl, Ethyl, n-Propyl, Isopropyl, n-Butyl, Isobutyl, sec-Butyl, tert-Butyl, Allyl, Propargyl, Methoxy, Ethoxy, n-Propoxy, Isopropoxy, n-Butoxy, Isobutoxy, sec-Butoxy, Allyloxy, Methoxycarbonylmethoxy, Ethoxycarbonylmethoxy, Methoxycarbonylethoxy, Ethoxycarbonylethoxy, Methylamino, Ethylamino, n-Propylamino, Isopropylamino, n-Butylamino, Isobutylamino, sec-Butylamino, tert-Butylamino, Dimethylamino, Acetylamino, Propionylamino, Methoxycarbonylamino, Ethoxycarbonylamino, Methylsulfonylamino, Ethylsulfonylamino, für jeweils gegebenenfalls durch Nitro, Cyano, Fluor, Chlor, Brom, Methyl, Trifluormethyl, Methoxy, Trifluormethoxy, Methylthio oder Trif luormethylthio substituiertes Phenyl, Benzyl, Phenoxy, Benzyloxy, Phenylamino, Benzylamino, N-Methyl-N-phenylamino, Pyridylamino, Pyrimidylamino, Pyridylcarbonylamino, Phenylcarbonylamino, Furylcarbonylamino, Thienylcarbonylamino oder Phenylsulfonylamino steht,

$R^{12}$ für Wasserstoff, Fluor, Chlor, Cyano, Carboxyl, $C_1$-$C_4$-Alkoxycarbonyl, $C_1$-$C_4$-Alkylcarbonylamino, Dimethoxyphosphoryl oder Diethoxyphosphoryl steht und

$R^{13}$ für Formyl, Cyano, Carboxyl, Hydroxymethyl, Carbamoyl, für einen jeweils gegebenenfalls durch Fluor, Chlor, Carboxy oder $C_1$-$C_4$-Alkoxycarbonyl substituierten Rest der Reihe $C_1$-$C_4$-Alkoxycarbonyl, $C_5$-$C_6$-Cycloalkyloxycarbonyl, $C_1$-$C_4$-Alkylthiocarbonyl, $C_1$-$C_4$-Alkylaminocarbonyl, $C_5$-$C_6$-Cycloalkylaminocarbonyl, Dimethylaminocarbonyl, $C_1$-$C_4$-Alkylaminocarbonyl-$C_1$-$C_4$-alkoxycarbonyl, Dimethylaminocarbonyl-$C_1$-$C_4$-alkoxycarbonyl,

N-Methyl-N-phenylaminocarbonyl-$C_1$-$C_4$-alkoxycarbonyl, oder für einen jeweils gegebenenfalls durch Methyl und/oder Ethyl substituierten Rest der Reihe Pyrrolidinylcarbonyl, Piperidinylcarbonyl, Morpholinylcarbonyl oder Piperazinylcarbonyl,
oder für jeweils gegebenenfalls durch Nitro, Cyano, Fluor, Chlor, Brom, Methyl, Trifluormethyl, Methoxy, Trifluormethoxy, Methylthio oder Trifluormethylthio substituierten Rest der Reihe Phenoxycarbonyl, Benzyloxycarbonyl, Phenylthiocarbonyl, Benzylthiocarbonyl, Phenylaminocarbonyl, Benzylaminocarbonyl, N-Methyl-N-phenylaminocarbonyl, Phenylhydrazinocarbonyl, für Phthalimidoxycarbonyl oder $R^{12}$ zusammen mit $R^{13}$ für die Gruppierungen $-COO-CH_2CH_2-$, $-COO-CH_2CH_2CH_2-$,

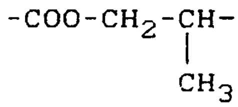
oder
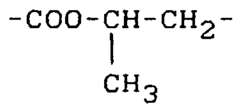

steht,

Y für Sauerstoff oder Imino (NH) steht und

Z für Stickstoff oder eine CH-Gruppierung steht.

Eine besonders interessante Untergruppe bilden die Verbindungen der Formel (I), worin

$R^1$ für Hydroxy, Methoxy, Ethoxy, Propoxy, Propargyloxy, Cyanomethoxy, Methylthioethoxy, Methoxycarbonylmethylamino, Methoxycarbonyl-ethylamino, 4-Chlorphenylethylamino, Methylsulfonylamino, Phenylsulfonylamino oder 2,2-Dimethylhydrazino steht,

$R^2$ für Wasserstoff, Fluor, Chlor, Methyl oder Ethyl steht,

$R^3$ für Wasserstoff, Methyl oder Ethyl steht,

$R^4$ für Wasserstoff, Methyl, Ethyl, Propyl oder Isopropyl steht,

$R^5$ für Wasserstoff, Methyl oder Ethyl steht,

$R^6$ für Wasserstoff, Methyl oder Ethyl steht,

$R^7$ für Wasserstoff, Methyl oder Ethyl steht,

$R^8$ für Wasserstoff oder Methyl steht,

$R^9$ für Wasserstoff, Methyl oder Methoxy steht,

$R^{10}$ für Methyl oder Methoxy steht,

X für Sauerstoff steht,

Y für Sauerstoff steht und

Z für Stickstoff oder die CH-Gruppe steht,

d.h. die freien Carbonsäuren ($R^1$ = OH), bestimmte Ester und Amide dieser Säuren sowie deren Alkalimetallsalze, insbesondere die Natrium- und Kaliumsalze.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die in der Definition der erfindungsgemäßen Verbindungen aufgeführten aliphatischen Kohlenwasserstoffreste (z.B. Alkyl, Alkenyl, Alkinyl), auch in Kombination mit Heteroatomen (z.B. in Alkoxy, Alkylthio, Alkylamino) oder in Zusammensetzung wie z.B. Halogenalkyl, Halogenalkoxy, sind jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Verwendet man für das erfindungsgemäße Herstellungsverfahren beispielsweise 2-Hydroxy-5-methylbicyclo[3.1.0]hexan-1-carbonsäure-ethylester und 2-Chlor-4,6-dimethoxy-s-triazin als Ausgangsstoffe, so kann der Reaktionsablauf durch das folgende Formelschema wiedergegeben werden:

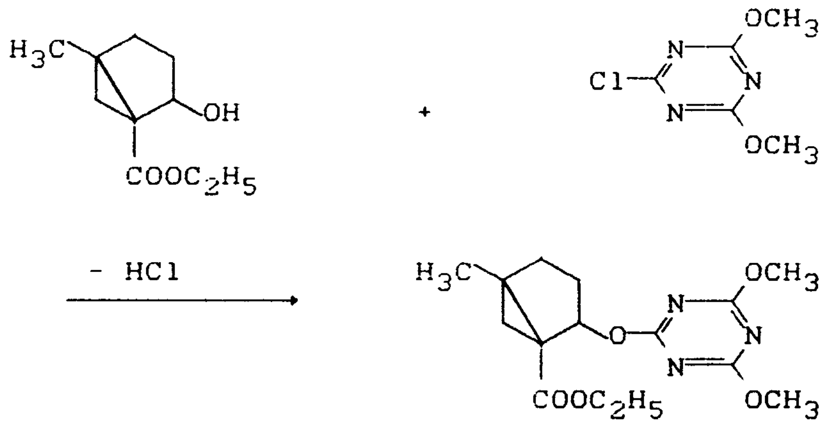


Die beim erfindungsgemäßen Verfahren zur Herstellung von Verbindungen der Formel (I) als Ausgangsstoffe zu verwendenden Bicyclo[3.1.0]hexane sind durch die obige Formel (II) allgemein definiert.

In Formel (II) haben $R^1$ bis $R^8$, X, Y und Z vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ bis $R^8$, X, Y bzw. Z angegeben wurden.

Die Bicyclo[3.1.0]hexane der Formel (II) sind noch nicht in der Literatur beschrieben worden, sondern sind neu und sind als solche auch Gegenstand dieser Erfindung.

Die Methoden zur Herstellung der Verbindungen der Formel (II) sind grundsätzlich bekannt und richten sich im einzelnen nach den gewünschten Substituenten:

(a) So erhält man die Verbindungen der Formel (II), worin

| | |
|---|---|
| $R^1$ | die oben bei Formel (I) genannten Bedeutungen - außer H, OH und Alkyl-hat, |
| $R^2$-$R^7$ | die obengenannten Bedeutungen haben, |
| $R^8$ | für H steht, |
| X | für 0 steht und |
| Y | für 0 steht, |

(d.h. die Ester, Thioester und Amide), wenn man die entsprechenden bicyclischen Ketone der Formel (IV)

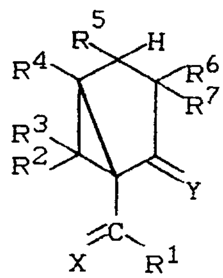

(IV)

worin

$R^1$ bis $R^7$, X und Y die vorgenannten Bedeutungen haben,

in an sich bekannter WEise mit einem komplexen Metallhydrid (wie z.B. Natriumborhydrid [$NaBH_4$] oder Lithiumaluminiumhydrid [$LiAlH_4$]) in einem geeigneten Lösungsmittel (z.B. einem Alkohol, wie Methanol, Ethanol, Isopropanol oder Butanol, oder einem Ether, wie beispielsweise Diethylether oder Tetrahydrofuran) bei Temperaturen zwischen 0 °C und 20 °C reduziert.

(b) Verbindungen der Formel (II), worin

| | |
|---|---|
| $R^1$ bis $R^7$, X und Y | die bei (a) genannten Bedeutungen haben und |
| $R^8$ | für Alkyl oder Aryl steht, |

können hergestellt werden, indem man die bicyclischen Ketone der Formel (IV) in an sich bekannter Weise mit geeigneten Alkyl- bzw. Aryl-Metallverbindungen umsetzt, z.B. mit Alkyl-Lithium- oder Aryl-Lithium-Verbindungen (wie beispielsweise n-Butyllithium oder Phenyllithium) oder mit den entsprechenden Grignard-Verbindungen.

(c) Verbindungen der Formel (II), worin

$R^1$ bis $R^7$, X und Y die bei (a) genannten Bedeutungen haben und

$R^8$ für Cyano oder Alkoxycarbonylalkyl steht,

können ebenfalls in an sich bekannter Weise direkt aus den Ketonen (IV) hergestellt werden: und zwar die Cyanhydrine ($R^8$ = CN) z.B. durch Umsetzung der Ketone mit Blausäure (HCN) oder mit einem Trialkylsilylcyanid, z.B. Trimethylsilylcyanid [$(CH_3)_3Si$-CN], und anschließende partielle Hydrolyse, und die Esterderivate ($R^8$ = Alkoxycarbonylethyl) durch Reformatzki-Synthese, d.h. Umsetzung der Ketone mit Halogenfettsäureestern (wie z.B. Bromessigsäureestern) und Zink.

(d) Verbindungen der Formel (II), worin

X für N-$R^{11}$ oder

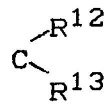

steht,

erhält man aus den entsprechenden Carbonylverbindungen (X = O) in an sich bekannter Weise durch Umsetzung mit Amino- bzw. Methylen-Verbindungen der Formel

$H_2X$, worin X für N-$R^{11}$ oder

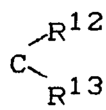

steht

(vgl. z.B. EP-A-451.653 ≙ US-Patent 5.185.026 und EP-A-459.243 ≙ US-Patent 5.167.693).

(e) Verbindungen der Formel (II), worin

X für O und $R^1$ für H steht (d.h. Aldehyde),

könnmen auf konventionelle Weise z.B. aus den entsprechenden Estern ($R^1$ = Alkoxy) durch Reduktion zu den primären Alkoholen und deren Oxidation zu den Aldehyden hergestellt werden.

(f) Verbindungen der Formel (II), worin X für S steht, können ebenfalls auf konventionelle Weise hergestellt werden, entweder auf Stufe der Verbindungen (II) mit X = O durch O/S-Austausch oder aus bereits schwefelhaltigen Vorstufen (s. unten).

(g) Verbindungen der Formel (II), worin Y für S steht, können durch analoge Reduktion aus den entsprechenden bicyclischen Thioxo-Verbindungen der Formel (IV), mit Y = S, hergestellt werden. Die letztgenannten Verbindungen erhält man wiederum durch O/S-Austausch, entweder durch Umsetzungen der Ketone (IV) oder der als Ausgangsprodukte zu verwendenden $\beta$-Ketoester (s. unten) mit dem sogenannten Lawesson-Reagenz (vgl. Bull. Soc. Chim. Belg. 1978, Seiten 223, 229, 299 und 525).

(h) Verbindungen der Formel (II), worin Y für NH steht, können aus den entsprechenden Ketonen (IV) hergestellt werden durch reduktive Aminierung z.B. mittels Natrium-cyanoboranat ($Na[BH_3CN]$) in Gegenwart von Ammoniumacetat (vgl. T. Sasaki, J. Chem. Soc., Perkin Trans. 1983, S. 3027).

(i) Aus den gemäß (h) erhältlichen Aminoverbindungen (II), mit Y = NH, lassen sich die N-Alkylverbindungen (II), mit Y = N-Alkyl, herstellen durch anschließende Alkylierung, z.B. mittels Alkylhalogeniden oder -sulfaten in Gegenwart von Butyllithium/Lithium-Diisopropylamid.

(j) Verbindungen der Formel (II), worin

X für O und $R^1$ für Alkyl steht (d.h. Alkylketone),

können ebenfalls auf konventionelle Weise, z.B. aus den entsprechenden Säuren ($R^1$ = OH) durch reduktive Alkylierung mittels 2 Moläquivalenten Alkyllithium pro Mol Säure in Gegenwart eines Ethers, wie z.B. Diethylether, hergestellt werden (vgl. z.B. Organic Reactions Vol. 18 (1970), Seiten 1 bis 97).

(k) Verbindungen der Formel (II), worin

X für O und $R^1$ für OH steht (d.h. die freien Säuren),

lassen sich am einfachsten durch Hydrolyse der zunächst erhaltenen entsprechenden Ester ($R^1$ = Alkoxy, Aryloxy, etc.) unter üblichen Bedingungen herstellen.

Die bicyclischen Ketone der Formel (IVa),

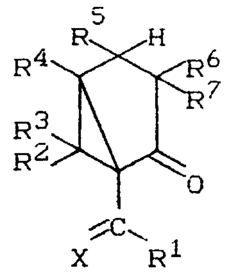

(IVa)

worin

$R^1$ bis $R^7$ und X die oben bei (a) genannten Bedeutungen haben,

- ausgenommen die drei folgenden Verbindungen:

2-Keto-6,6-dimethyl-bicyclo[3.1.0]hexan-1-carbonsäure-methylester (vgl. J. Org. Chem. 48, S 2076 bis 2084 (1983)); sowie 2-Keto-6-methyl- und 2-Keta-6-n-propyl-bicyclo[3.1.0]-hexan-1-carbonsäure-ethylester (vgl. Bull. Chem. Soc. Japan 54, p. 2154-2160 (1981)), -

sind neu, Die neuen Verbindungen der Formel (IVa) sind gleichfalls Gegenstand der vorliegenden Erfindung.

Es wurde überraschend gefunden. daß die bicyclischen Ketone der Formel (IV), worin

| | |
|---|---|
| $R^1$ | für Alkoxy steht, |
| $R^4$ | mit Ausnahme von Wasserstoff die obengenannten Bedeutungen hat, die Reste |
| $R^2$, $R^3$, $R^5$, $R^6$ und $R^7$ | die obengenannten Bedeutungen haben und |
| X | für Sauerstoff steht, |

nach einem neuen erfinderischen Verfahren hergestellt werden können, welches dadurch gekennzeichnet ist, daß man - nach folgendem Reaktionsschema -

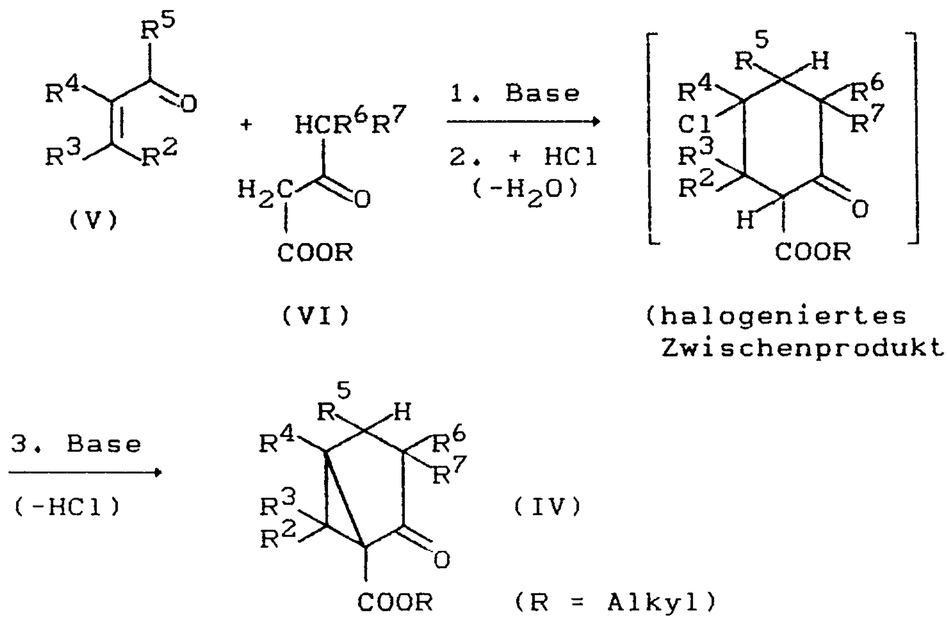


($\alpha$) eine $\alpha.\beta$-ungesättigte Carbonylverbindung der Formel (V) mit einem $\beta$-Keto-ester der Formel (VI), wobei in den Formeln (V) und (VI)

| | |
|---|---|
| $R^1$ | für Alkoxy steht, |
| $R^4$ | mit Ausnahme von Wasserstoff die obengenannten Bedeutungen hat, die Reste |
| $R^2$, $R^3$, $R^5$, $R^6$ und $R^7$ | die obengenannten Bedeutungen haben und |
| X | für Sauerstoff steht, |

in etwa äquimolaren Mengen zunächst in Gegenwart einer starken Base [wie z.B. NaH, $NaNH_2$, LiNCH-

$(CH_3)_2$ oder eines Alkoholats wie z.B. $NaOCH_3$, $NaOC_2H_5$ oder $KOC(CH_3)_3$] und in Gegenwart eines polaren Lösungsmittels (z.B. Ethanol, Methanol, Tetrahydrofuran, Dimethylsulfoxid, Acetonitril, Dioxan, Dimethylformamid; vorzugsweise Ethanol) bei Temperaturen zwischen -50 °C und +100 °C, vorzugsweise zwischen -40 °C und +80 °C, besonders bevorzugt zwischen 0 °C und +40 °C, umsetzt,

($\beta$) das Reaktionsprodukt anschließend - ohne Zwischenisolierung - bei etwa 0 °C mit wasserfreiem Chlorwasserstoff (durch Sättigen der Lösung mit HCl-Gas) umsetzt und

($\gamma$) schließlich das hierbei gebildete halogenierte Zwischenprodukt, z.B. durch Destillation, isoliert und dieses erneut mit einer Base (ausgewählt z.B. aus den unten, in Zusammenhang mit dem Hauptverfahren zur Herstellung der Endprodukte (I) aufgeführten Säureakzeptoren; besonders bevorzugt mit 1,5-Diazabicyclo[5.4.0]undec-5-en(DBN), welches auch als Verdünnungsmittel dienen kann) bei Temperaturen zwischen 0 °C und 150 °C, vorzugsweise zwischen 20 °C und 120 °C, besonders bevorzugt zwischen 50 °C und 100 °C, zur Umsetzung bringt, wobei unter Halogenwasserstoff-Eliminierung die Bildung des Dreiringes und damit des Bicyclo[3.1.0]hexangerüstes erfolgt.

Dieses neue Verfahren zur Herstellung der bicyclischen Keto-ester ist deshalb überraschend und als solches erfinderisch, weil nach dem Stand der Technik (vgl. F.M. Hauser et. al., SYNTHESIS 1980, S. 814-815) zu erwarten war, daß bei dieser Umsetzung die mit (IV) isomeren Cyclohexenon-Ester (A) gebildet würden:

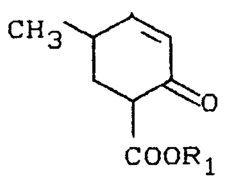

(A)

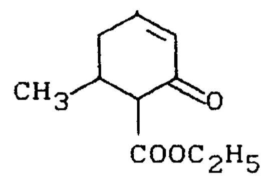

(B)

Die Verbindungen (A) waren das ursprüngliche Syntheseziel, da die vergleichbare Umsetzung von Crotonaldehyd mit Acetessigsäureethylester nach F.M. Hauser et. al. (l.c.) zum Cyclohexenon-Ester (B) führt.

Verwendet man als Ausgangsstoff der Formel (V) Methacrolein und als Ausgangsstoff der Formel (VI) Acetessigsäuremethylester, so läßt sich das Verfahren zur Herstellung der bicyclischen Keto-ester (IV) durch folgendes Formelschema veranschaulichen:

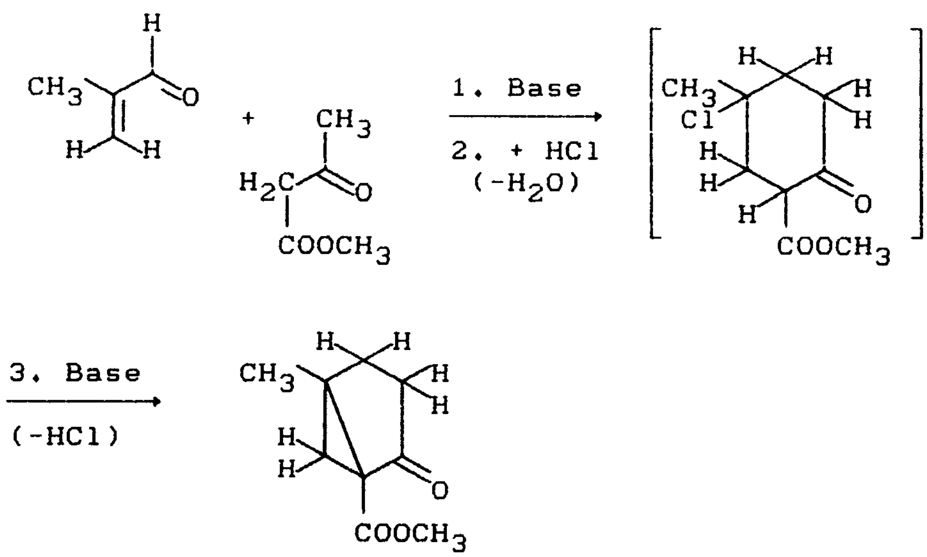


Bicyclische Keto-ester der obigen Formel (IV), worin

| | |
|---|---|
| $R^1$ | für Alkyl steht, |
| $R^4$ | für Wasserstoff steht, |
| $R^2$ bis $R^7$ | die obengenannten Bedeutungen haben und |

X für Sauerstoff steht,

können nach literaturbekannten Methoden hergestellt werden (vgl. die oben bereits zitierten Literaturstellen J. Org. Chem. 48, S. 2076-2084 (1983) und Bull. Chem. Soc. Japan Vol. 54, S. 2154-2160 (1981)).

Das Bicyclo[3.1.0]hexan-Ringgerüst mit unterschiedlichen Substituenten, welches charakteristisch ist für die Zwischenprodukte vom Typ (IV) bzw. (II), kann auch nach weiteren bereits bekannten Methoden aufgebaut werden; die Substituenten können entweder durch Wahl geeigneter Ausgangsprodukte oder gegebenenfalls durch nachfolgende Umwandlung durch konventionelle Methoden festgelegt werden. Beispielhaft hierfür seien die Carben-Addition bzw. Methylen-Übertragung auf geeignete Cyclopenten-Derivate genannt.

So läßt sich z.B. nach dem Verfahren von E.J. Corey et al. die Methylengruppe ($-CH_2-$) unter Bildung eines Cyclopropanringes spezifisch an die konjugierte C=C-Doppelbindung eines Cyclopentenoncarbonsäureesters vom Typ (C) addieren, indem man nach folgendem Reaktionsschema:

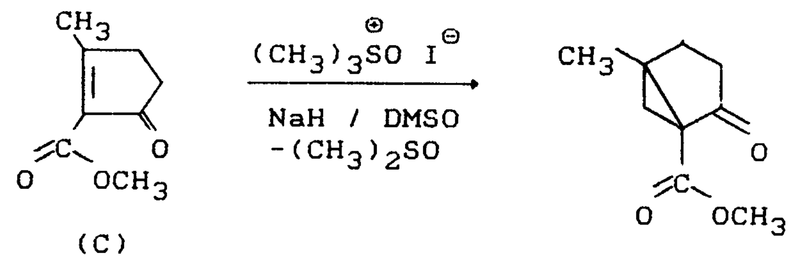


(C)

beispielsweise 2-Keto-5-methyl-cyclopentenyl-1-carbonsäuremethylester (C) mit Dimethyloxosulfonium-Methylid

$$[(CH_3)_2\overset{\oplus}{S}O-\overset{\ominus}{C}H_2]$$

- erzeugt aus Trimethyloxosulfonium-Iodid

$$[(CH_3)_3\overset{\oplus}{S}OI^{\ominus}]$$

durch Deprotonierung mittels starker Basen wie Natriumhydrid (NaH) in Dimmethylsulfoxid (DMSO) oder Tetrahydrofuran (THF) - umsetzt (vgl. E.J. Corey und M. Chaykovsky, J. Amer. Chem, Soc. Vol. 87 (1965), Seiten 1353 bis 1364).

Die bicyclischen Keto-ester der obigen Formel (IV), worin $R^1$ die übrigen der obengenannten Bedeutungen hat (d.h. die freien Säuren, die Aldehyde und Ketone, ferner ungesättigte, aromatische und heteroaromatische Ester, Thioester und Amide), können nach konventionellen Verfahren aus den primär hergestellten Alkylestern ($R^1$ = Alkoxy) gewonnen werden; diese konventionellen Verfahren zur Umwandlung der funktionellen Gruppen ($-C(X)R^1$) sind bereits oben im Zusammenhang mit den Zwischenprodukten der Formel (II) aufgeführt worden.

Zusammenfassend kann zur Synthese der Zwischenprodukte der Formeln (II) und (IV) festgestellt werden, daß nur der Aufbau des Bicyclo[3.1.0]hexan-Systems durch die oben beschriebene neuartige Kondensations- bzw. Additions-/Eliminierungsreaktion erfinderisch ist, und daß alle anschließenden Abwandlungen der Grundstruktur (IV) bzw. (II) mittels prinzipiell bekannter Methoden möglich sind, Die jeweiligen Reaktionsbedingungen (z.B. Wahl der geeigneten Lösungsmittel, Hilfsstoffe, Mengenverhältnisse, Temperatur- und Druckbereiche) lassen sich im Rahmen der üblichen Verfahrensweisen leicht ermitteln.

Die beim erfindungsgemäßen Verfahren zur Herstellung der Bicyclo[3.1.0]hexane der Formel (I) weiterhin als Ausgangsstoffe zu verwendenden Azine sind durch Formel (III) definiert In Formel (III) haben $R^9$, $R^{10}$ und Z vorzugsweise bzw. insbesondere diejenigen Bedeutungen, die bereits oben im Zusammenhang mit der Beschreibung der erfindungsgemäßen Verbindungen der Formel (I) als bevorzugt bzw. als insbesondere bevorzugt für $R^9$, $R^{10}$ bzw. Z angegeben wurden; weiter steht in Formel (III)

Q vorzugsweise für Fluor, Chlor, Brom oder $C_1$-$C_4$-Alkylsulfonyl, insbesondere für Chlor oder Methylsulfonyl.

Als Beispiele für die Ausgangsstoffe der Formel (III) seien genannt:

2-Chlor-4,6-dimethyl-pyrimidin, 2-Chlor-4-methyl-6-methoxy-pyrimidin, 2-Chlor-4,6-dimethoxy-pyrimidin, 2-Chlor-4-methylpyrimidin, 2-Chlor-4,6-dimethyl-s-triazin, 2-Chlor-4-methoxy-6-methyl-s-triazin, 2-Chlor-4,6-dimethoxy-s-triazin, sowie 2-Methylsulfonyl-4,6-dimethylpyrimidin, 2-Methylsulfonyl-4,6-dimethoxy-pyrimidin und 2-Methylsulfonyl-4-methyl-pyrimidin.

Die Azine der Formel (III) sind bekannt und/oder können nach an sich bekannten Verfahren hergestellt werden (vgl. J. Chem. (1957), S.1830, S.1833; J. Org. Chem. 26 (1961), S. 792; US-P 3308119; US-P 4711959).

Das erfindungsgemäße Verfahren zur Herstellung der neuen Verbindungen der Formel (I) wird vorzugsweise unter Verwendung von Verdünnungsmitteln durchgeführt. Als Verdünnungsmittel kommen dabei inerte organische Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methylisobutyl-keton, Ester wie Essigsäuremethylester und - ethylester, Nitrile wie z.B. Acetonitril und Propionitril, Amide wie z.B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei dem erfindungsgemäßen Verfahren alle üblicherweise für derartige Umsetzungen verwendbaren Säurebindemittel eingesetzt werden. Vorzugsweise infrage kommen Alkalimetallhydroxide, wie z.B. Natrium- und Kaliumhydroxid, Erdalkalihydroxide wie z.B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo[5,4,0]-undec-7-en (DBU) und 1,4-Diazabicyclo-[2,2,2]octan (DABCO).

Die Reaktionstemperaturen können bei dem erfindungsgemäßen Verfahren in einem größeren Bereich variiert werden Im allgemeinen arbeitet man bei Temperaturen zwischen 0 °C und 150 °C, vorzugsweise bei Temperaturen zwischen 10 °C und 100 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührte Die Aufarbeitung erfolgt bei dem erfindungsgemäßen Verfahren jeweils nach üblichen Methoden (vergleiche die Herstellungsbeispiele).

Die erfindungsgemäßen Wirkstoffe der Formel (I) können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich zur Bekämpfung von monokotylen und dikotylen Unkräutern, insbesondere in dikotylten Kulturen, sowohl im Vorauflauf- als auch im Nachauf lauf-Verfahren.

Die Wirkstoffe können in die üblichen Formulierungen überführt werden, wie Lösungen, Emulsionen, Spritzpulver, Suspensionen, Pulver, Staubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl. Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

Herstellungsbeispiele:

Beispiel 1

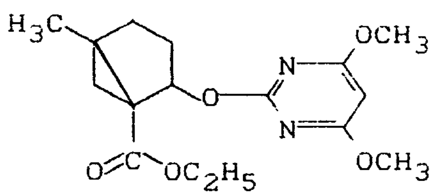

Eine Mischung aus 1,4 g (6,5 mMol) 4,6-Dimethoxy-2-methylsulfonyl-pyrimidin, 1,2 g (6,5 mMol) 2-Hydroxy-5-methyl-bicyclo-[3.1.0]-hexan-1-carbonsäure-ethylester, 1,1 g (5,5 mMol) Kaliumcarbonat und 20 ml Acetonitril wird 12 Stunden unter Rückfluß erhitzt und anschließend eingeengt. Der Rückstand wird mit Wasser / Essigsäureethylester ausgeschüttelt, die organische Phase abgetrennt, mit Natriumsulfat getrocknet und filtriert. Vom Filtrat wird das Lösungsmittel im Wasserstrahlvakuum abdestilliert.

Man erhält 1,1 g (52 % der Theorie) 2-(4,6-Dimethoxypyrimidin-2-yloxy)-5-methyl-bicyclo[3.1.0]hexan-1-carbonsäure-ethylester in Form eines Öls.

$^{1}$H-NMR-Spektrum (300 MHz; Tetramethylsilan als innerer Standard = TMS): $\delta$ = 3,85 ppm (s, 2 Methoxy-Gruppen).

Für alle nachfolgend angegebenen NMR-Daten gelten die gleichen Meßbedingungen (300 MHz; TMS).

Beispiel 2

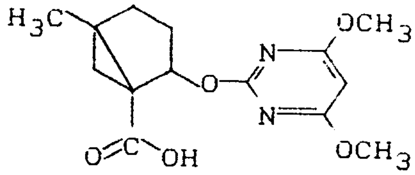

2-(4,6-Dimethoxy-pyrimidin-2-yloxy)-5-methylbicyclo[3.1.0]hexan-1-carbonsäure vom Schmelzpunkt 186 °C, hergestellt durch Esterhydrolyse des entsprechenden Ethylesters (Beispiel 1) unter üblichen Bedingungen.

Beispiel 3

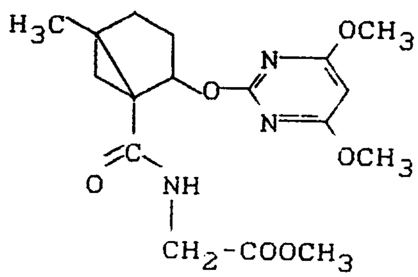

1 g (3,4 mMol) 2-(4,6-Dimethoxy-pyrimidin-2-yloxy)-5-methyl-bicyclo[3.1.0]hexan-1-carbonsäure wird in 50 ml Dichlormethan gelöst und mit 2,1 g Triethylamin versetzt. Bei -5 °C läßt man 0,53 g (3,4 mMol) Phosphoroxychlorid, gelöst in 5 ml Dichlormethan, zutropfen und rührt 5 Minuten nach. Unter weiterem Rühren wird dann zu der Lösung des Säurechlorids eine Lösung von 0,9 g (7,1 mMol) Aminoessigsäuremethylester (Glycinsäuremethylester) in 5 ml Dichlormethan zugegeben. Man läßt dann das Reaktionsgemisch innerhalb von 15 Minuten Raumtemperatur erreichen. Man trennt anschließend die organische Phase ab, wäscht diese dreimal mit Wasser, trocknet über Magnesiumsulfat und dampft ein. Der verbleibende Rückstand wird mittels (Flush)Chromatographie gereinigt. Man erhält 2-(4,6-Dimethoxy-pyrimidin-2-yloxy)-5-methyl-bicyclo[3.1.0]hexan-1-carbonsäure-N-(methoxycarbonylmethyl)-amid in Form eines Öls.

$^1$H-NMR-Spektrum:

$\delta$ = 3,58 ppmm (s; $-COOCH_3$).

Beispiel 4

2(4,6-Dimethoxy-pyrimidin-2-yloxy)-5-methylbicyclo[3.1.0]hexan-1-carbonsäure-Natriumsalz vom Schmelzpunkt 125 °C (Zers. = unter Zersetzung), hergestellt durch Neutralisation der Carbonsäure (Beispiel 2) unter üblichen Bedingungen.

Beispiel 5

2-(4,6-Dimethoxy-pyrimidin-2-yloxy)-5-methylbicyclo[3.1.0]hexan-1-carbonsäure-Kaliumsalz vom Schmelzpunkt 165 °C (Zers.), hergestellt analog zu Beispiel 4.

Beispiel 6

2-(4,6-Dimethoxy-pyrimidin-2-yloxy)-5-methyl-bicyclo[3.1.0]hexan-1-carbonsäure-methylester vom Schmelzpunkt 76 °C, hergestellt analog zu Beispiel 1.

Beispiel 7

2-(4,6-Dimethoxy-pyrimidin-2-yloxy)-5-methylbicyclo[3.1.0]hexan-1-carbonsäure-n-propylester in Form eines Öls, hergestellt analog zu Beispiel 1.

$^1$H-NMR-Spektrum:

δ = 0,86 ppm (t; $-COOCH_2CH_2\underline{CH_3}$).

Beispiel 8

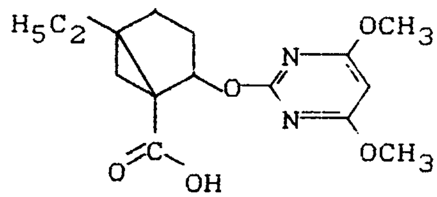

2-(4,6-Dimethoxy-pyrimidin-2-yloxy)-5-ethyl-bicyclo[3.1.0]hexan-1-carbonsäure in Form eines Öls, hergestellt analog zu den Beispielen 1 und 2.

$^1$H-NMR-Spektrum:

δ = 3,85 ppm (s; 2 $O\underline{CH_3}$).

Beispiel 9

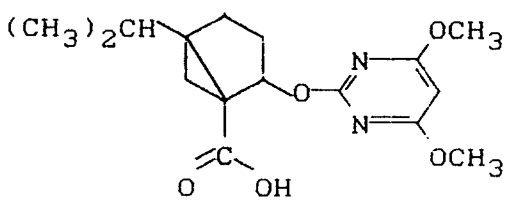

2-(4,6-Dimethoxy-pyrimidin-2-yloxy)-5-isopropyl-bicyclo[3.1.0]hexan-1-carbonsäure vom Schmelzpunkt 170 °C, hergestellt analog zu den Beispielen 1 und 2.

Beispiel 10

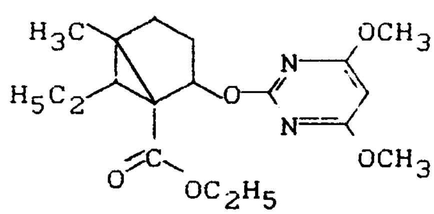

2-(4,6-Dimethoxy-pyrimidin-2-yloxy)-5-methyl-6-ethylbicyclo[3.1.0]hexan-1-carbonsäure-ethylester (als Isomerengemisch) in Form eines Öls, hergestellt analog zu Beispiel 1.

$^1$H-NMR-Spektrum:

δ = 6,12 (t;

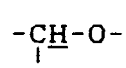

in 2-Position).

<u>Beispiel 11</u>

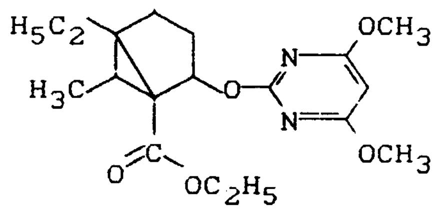

2-(4,6-Dimethoxy-pyrimidin-2-yloxy)-5-ethyl-6-methyl-bicyclo[3.1.0]hexan-1-carbonsäure-ethylester (als Isomerengemisch) in Form eines Öls, hergestellt analog zu Beispiel 1.

$^1$H-NMR-Spektrum:

$\delta$ = 6.10 ppm (t;

$$-C\underline{H}-O-$$

in 2-Position).

<u>Beispiel 12</u>

2-(4,6-Dimethoxy-pyrimidin-2-yloxy)-5-ethyl-6-methylbicyclo[3.1.0]hexan-1-carbonsäure (als Isomerengemisch) in Form eines Öls, hergestellt durch Esterhydrolyse des entsprechenden Ethylesters (Beispiel 11) unter üblichen Bedingungen.

$^1$H-NMR-Spektrum:

$\delta$ = 5.98 ppm (t;

$$-C\underline{H}-O-$$

in 2-Position).

<u>Beispiel 13</u>

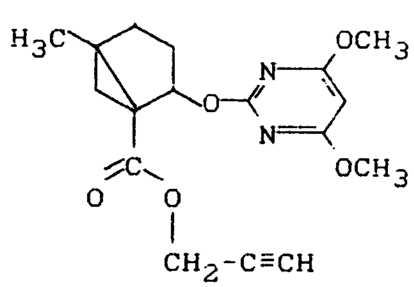

2-(4,6-Dimethoxy-pyrimidin-2-yloxy)-5-methyl-bicyclo[3.1.0]hexan-1-carbonsäure-propargylester vom Schmelzpunkt 170 °C, hergestellt analog zu Beispiel 3 über das Säurechlorid.

Beispiel 14

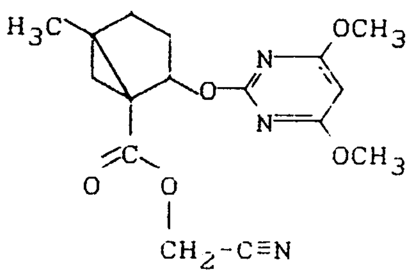

2(4,6-Dimethoxy-pyrimidin-2-yloxy)-5-methyl-bicyclo[3.1.0]hexan-1-carbonsäure-cyanomethylester in Form eines Öls, hergestellt durch Umsetzung des Natriumsalzes der Carbonsäure (Beispiel 4) mit handelsüblichem Chloracetonitril.

$^1$H-NHR-Spektrum:

δ = 4,68 und 4,80 ppm (AB-System; -COO-$\underline{CH_2}$-CN).

Beispiel 15

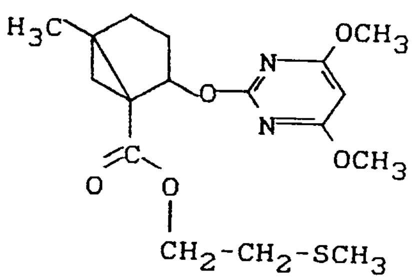

2-(4,6-Dimethoxy-pyrimidin-2-yloxy)-5-methyl-bicyclo[3.1.0]hexan-1-carbonsäure-(2-methylthioethyl)-ester in Form eines Öls, hergestellt analog zu Beispiel 3.

$^1$H-NMR-Spektrum:

δ = 2.09 ppm (s; -$CH_2CH_2$-S$\underline{CH_3}$).

Beispiel 16

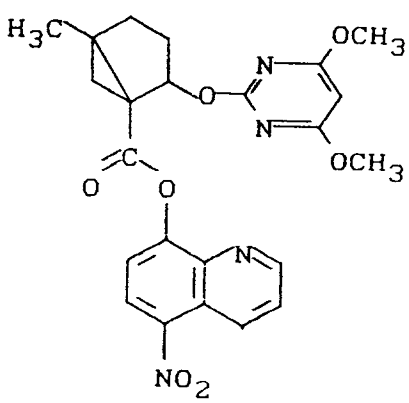

2-(4,6-Dimethoxy-pyrimidin-2-yloxy)-5-methyl-bicyclo[3.1.0]hexan-1-carbonsäure-(5-nitro-8-chinolyl)-ester in Form eines Öls, hergestellt analog zu Beispiel 3.

$^1$H-NMR-Spektrum:
δ = 3,83 ppm (s; 2 -O$CH_3$).

Beispiel 17

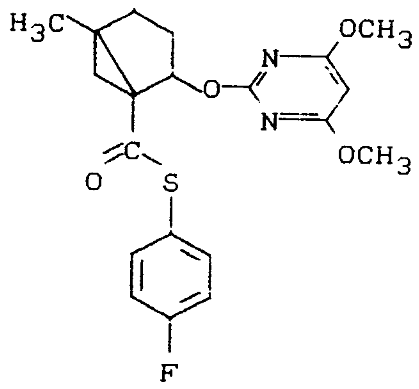

2-(4,6-Dimethyloxy-pyrimidin-2-yloxy)-5-methy-bicyclo[3.1.0]hexan-1-thiocarbonsäure-S-(4-fluorphenyl)-ester vom Schmelzpunkt 89 ° C, hergestellt analog zu Beispiel 3.

Beispiel 18

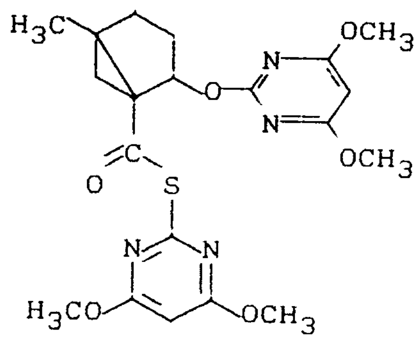

2-(4,6-Dimethoxy-pyrimidin-2-yloxy)-5-methyl-bicyclo[3.1.0]hexan-1-thiocarbonsäure-S-(4,6-dimethoxy-pyrimidin-2-yl)-ester vom Schmelzpunkt 146 ° C, hergestellt analog zu Beispiel 3.

Beispiel 19

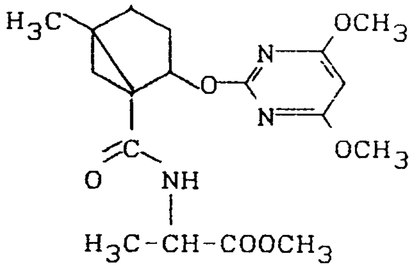

2-(4,6-Dimethoxy-pyrimidin-2-yloxy)-5-methyl-bicyclo[3.1.0]hexan-1-carbonsäure-N-(1-methoxycarbonyl-ethyl)amid in Form eines Öls, hergestellt analog zu Beispiel 3.

$^1$H-NMR-Spektrum:

$\delta$ = 3,93 ppm (s; 2 -$OCH_3$).

Beispiel 20

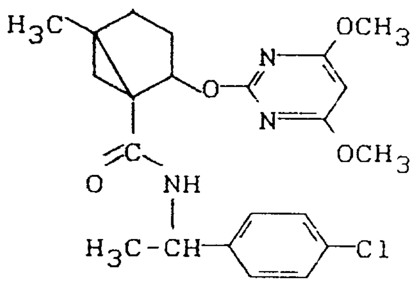

2-(4,6-Dimethoxy-pyrimidin-2-yloxy)-5-methyl-bicyclo[3.1.0]hexan-1-carbonsäure-N-[1-(4-chlorphenyl)-ethyl]-amid in Form eines Öls, hergestellt analog zu Beispiel 3.

$^1$H-NMR-Spektrum:

$\delta$ = 3,85 ppm (s; 2 $OCH_3$).

Beispiel 21

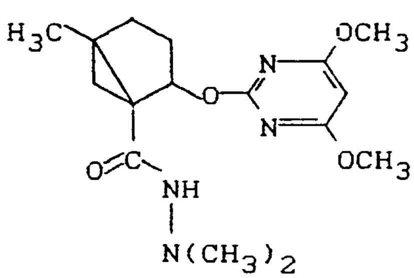

2-(4,6-Dimethoxy-pyrimidin-2-yloxy)-5-methyl-bicyclo[3.1.0]hexan-1-carbonsäure-(2,2-dimethyl)-hydrazid vom Schmelzpunkt 78 ° C, hergestellt analog zu Beispiel 3.

Beispiel 22

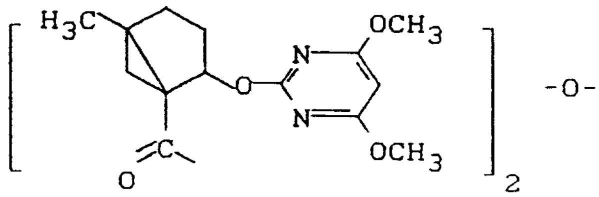

2-(4,6-Dimethoxy-pyrimidin-2-yloxy)-5-methylbicyclo[3.1.0]hexan-1-carbonsäure-anhydrid vom Schmelzpunkt 66 °C, hergestellt durch Umsetzung des Natriumsalzes der Carbonsäure (Beispiel 4) mit dem entsprechenden Säurechlorid.

Herstellung der Ausgangsverbindungen

Beispiel II-1

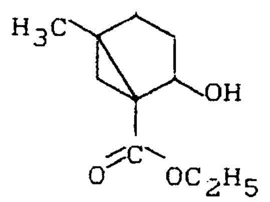

Zu 1,8 g (0,01 Mol) 2-Keto-5-methyl-bicyclo[3.1.0]hexan-1-carbonsäure-ethylester, gelöst in 20 ml Ethanol, werden bei Raumtemperatur 0,4 g (0,01 Mol) Natriumborhydrid gegeben, dann wird ca. 12 Stunden bei Raumtemperatur nachgerührt. Das Reaktionsgemisch wird in Wasser gegossen. Man extrahiert den Rückstand mit Diethylether, engt ein und chromatographiert das Rohprodukt an Kieselgel mit Hexan/Essigsäureethylester als Laufmittel.

Man erhält 1,5 g (82 % der Theorie) 2-Hydroxy-5-methyl-bicyclo[3.1.0]hexan-1-carbonsäure-ethylester in Form eines Öls.

$^1$H-NMR-Spektrum:

$\delta$ = 1,12 ppm (s, tert. Methylgruppe, in 5-Position)

Die folgenden Zwischenprodukte der Formel (II) können in analoger Weise hergestellt werden:

Beispiel II-2

$^1$H-NMR-Spektrum:

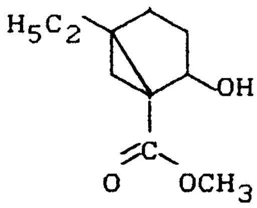

δ = 4,92 ppm (t; -CH-OH in 2-Position

Beispiel II-3

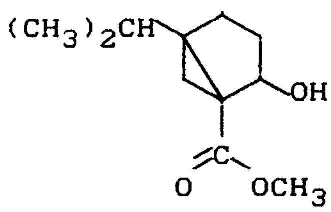

δ = 4,91 ppm (t; -CH-OH in 2-Position)

Beispiel II-4

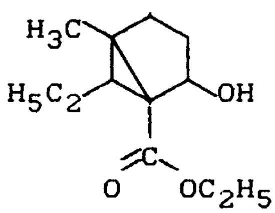

δ = 4,76 ppm (t; -CH-OH in 2-Position)

Beispiel II-5

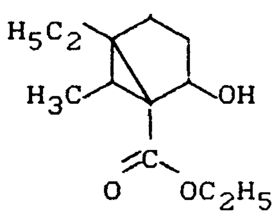

δ = 4,77 ppm (t; -CH-OH in 2-Position)

Beispiel IV-1

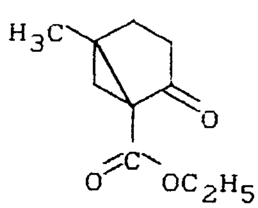

74 g (0,57 Mol) Acetessigsäureethylester gibt man zu einer Lösung von 0,5 g Natrium in 100 ml getrocknetem Ethanol, tropft 40 g (0,57 Mol) Methacrolein zu und rührt ca. 12 Stunden bei Raumtemperatur.

Die Mischung wird dann bei 0 °C mit trockenem HCl-Gas gesättigt und anschließend ca. 48 Stunden bei Raumtemperatur nachgerührt. Man entfernt das Lösungsmittel und destilliert den verbliebenen Rückstand im Vakuum. Die von 95 °C bis 105 °C / 0,013 mbar übergehenden Fraktionen werden vereinigt. Man erhält 32,3 g eines gelben Öls, das gelöst in 50 ml 1,5-Diazabicyclo[5.4.0]undec-5-en auf 80 °C erwärmt und 30 Minuten bei dieser Temperatur nachgerührt wird. Die so erhaltene Lösung wird in Wasser gegossen, mit Methylenchlorid extrahiert, anschließend mit 10%iger Salzsäure gewaschen, über Natriumsulfat getrocknet und eingeengt.

Man erhält 20,4 g 2-Keto-5-methyl-bicyclo[3.1.0]hexan-1-carbonsäure-ethylester in Form eines Öls.

$^1$H-NMR-Spektrum:

$\delta$ = 1,37 ppm (s; tert. Methylgruppe, in 5-Position)

Die folgenden Zwischenprodukte der Formel (IV) können in analoger Weise hergestellt werden:

Beispiel IV-2

$^1$H-NMR-Spektrum:

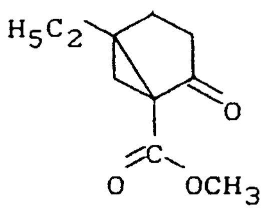

$\delta$ = 1,46 ppm und 1,98 ppm (AB-System; -C$\underline{H}_2$- in 6-Position)

Beispiel IV-3

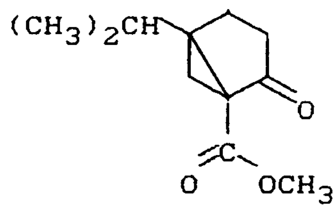

$\delta$ = 1,47 ppm und 1,98 ppm (AB-System; -C$\underline{H}_2$- in 6-Position)

Beispiel IV-4

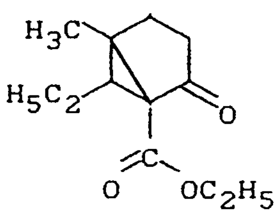

$\delta$ = 1,41 ppm (s; tert. Methylgruppe in 5-Position)

Beispiel IV-5

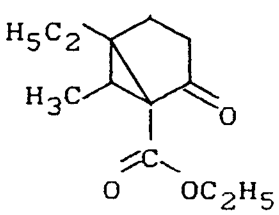

$\delta$ = 1,30 ppm (d; Methylgruppe in 6-Position)

Verwendungsbeispiele

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten;

O % = keine Wirkung (wie unbehandelte Kontrolle)
100 % = totale Vernichtung

In diesem Test zeigt z.B. die Verbindung 2 eine starke herbizide Wirksamkeit. So werden bei einer Aufwandmenge von 500 g/ha z.B. die Unkräuter Alopecurus, Bromus, Cyperus, Echinochloa, Veronica und Viola zu 90 bis 100 % vernichtet, bei gleichzeitig guter Verträglichkeit in Kulturpflanzen wie beispielsweise Soja.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:

O % = keine Wirkung (wie unbehandelte Kontrolle)
100 % = totale Vernichtung

Auch in diesem Test zeigt z.B. die Verbindung 2 eine starke herbizide Wirksamkeit. So werden bei einer Aufwandmenge von 500 g/ha z.B. die Unkräuter Bromus, Sorghum, Abutilon, Amaranthus, Sinapis und Veronica zu 90 bis 100 % vernichtet, bei gleichzeitig voller Verträglichkeit in Kulturpflanzen wie beispielsweise Soja.

## Patentansprüche

**1.** Substituierte Bicyclo[3.1.0]hexane der allgemeinen Formel (I)

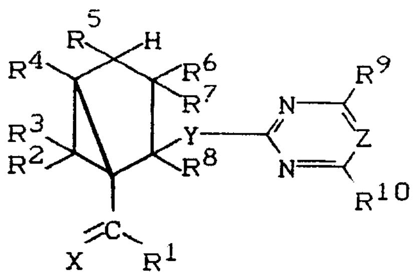

(I)

in welcher

$R^1$ für Wasserstoff, Hydroxy, Amino oder für einen jeweils gegebenenfalls substituierten Rest aus der Reihe Alkyl, Alkoxy, Alkenyloxy, Alkinyloxy, Aryloxy, Heteroaryloxy, Alkylthio, Arylthio, Heteroarylthio, Alkylamino, Dialkylamino, Alkylsulfonylamino, Arylsulfonylamino oder Hydrazino steht,

$R^2$ für Wasserstoff, Halogen, Alkyl, Alkoxy, Aryl oder Alkoxycarbonyl steht,

$R^3$ für Wasserstoff oder Alkyl oder - wenn $R^2$ Wasserstoff, Alkyl oder Halogen bedeutet - auch für Halogen steht,

$R^4$ für Wasserstoff, Halogen, Alkyl, Aryl oder Alkoxycarbonylalkyl steht,

$R^5$ für Wasserstoff, Alkyl oder Aryl steht,

$R^6$ für Wasserstoff, Hydroxy, Amino, Halogen, Cyano, Alkyl, Alkoxy, Aryl oder Alkoxycarbonyl steht,

$R^7$ für Wasserstoff oder Alkyl oder - wenn $R^6$ Wasserstoff, Alkyl oder Halogen bedeutet - auch für Halogen steht,

$R^8$ für Wasserstoff, Cyano, Alkyl, Aryl oder Alkoxycarbonylalkyl steht,

$R^9$ und $R^{10}$ gleich oder verschieden sind und für Wasserstoff, Halogen oder für einen jeweils gegebenenfalls substituierten Rest der Reihe Alkyl, Alkoxy, Alkylthio, Alkylamino, Dialkylamino, Aryl oder Aryloxy stehen,

X für Sauerstoff oder Schwefel oder - wenn $R^1$ Wasserstoff oder Alkyl bedeutet - auch für eine der nachstehenden Gruppierungen steht:

N-$R^{11}$ oder

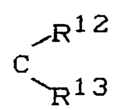

wobei

$R^{11}$ für Wasserstoff, Hydroxy, Amino oder für einen jeweils gegebenenfalls substituierten Rest der Reihe Alkyl, Alkenyl, Alkinyl, Alkoxy, Alkenyloxy, Alkoxycarbonylalkoxy, Alkylamino, Dialkylamino, Alkylcarbonylamino, Alkoxycarbonylamino, Alkylsulfonylamino, Aryl, Aralkyl, Aryloxy, Aralkyloxy, Arylamino, Aralkylamino, N-Alkyl-N-arylamino, Hetarylamino, Hetarylcarbonylamino, Arylcarbonylamino oder Arylsulfonylamino steht,

$R^{12}$ für Wasserstoff, Halogen, Cyano, Carboxyl, Alkoxycarbonyl, Alkylcarbonylamino oder Dialkoxyphosphoryl steht und

$R^{13}$ für Formyl, Cyano, Carboxyl, Hydroxymethyl, Carbamoyl oder für einen jeweils gegebenenfalls substituierten Rest der Reihe Alkoxycarbonyl, Cycloalkyloxycarbonyl, Alkylthiocarbonyl, Alkylaminocarbonyl, Cycloalkylaminocarbony, Dialkylaminocarbonyl, Alkylaminocarbonylalkoxycarbonyl, Dialkylaminocarbonylalkoxycarbonyl, Arylaminocarbonylalkoxycarbonyl, N-Alkyl-N-arylaminocarbonylalkoxycarbonyl, Pyrrolidinylcarbonyl, Piperidinylcarbonyl, Morpholinylcarbonyl, Piperazinylcarbonyl, Aryloxycarbonyl, Aralkyloxycarbonyl, Heterocyclylalkoxycarbonyl, Arylthiocarbonyl, Aralkylthiocarbonyl, Arylaminocarbonyl, Aralkylaminocarbonyl, N-Alkyl-N-arylaminocarbonyl, Arylhydrazinocarbonyl, Alkylhydrazinocarbonyl, Phthalimidoxycarbonyl oder $R^{12}$ zusammen mit $R^{13}$ für die Gruppierung

$-CO-O-(CH_2)_n-$ steht, wobei n für die Zahlen 1 bis 4 steht,

Y für Sauerstoff, Schwefel, Imino (NH) oder Alkylimino (N-Alkyl) steht, und

Z für Stickstoff oder die Gruppierung C-$R^{14}$ steht, worin $R^{14}$ für Wasserstoff, Halogen, Alkyl oder Alkoxy steht,

sowie Salze der freien Säuren ($R^1$ = OH) und die Anhydride der freien Säuren.

**2.** Verfahren zur Herstellung von substituierten Bicyclo[3.1.0]hexanen der Formel (I) gemäß Anspruch 1, dadurch gekennzeichnet, daß man Bicyclo[3.1.0]-hexane der Formel (II),

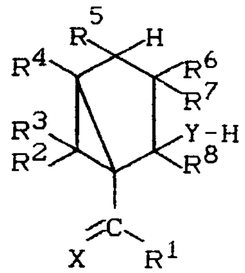

(II)

in welcher
$R^1$, $R^2$, $R^3$, $R^4$, $R^5$, $R^6$, $R^7$, $R^8$, X und Y die in Anspruch 1 angegebenen Bedeutungen haben, mit Azinen der Formel (III),

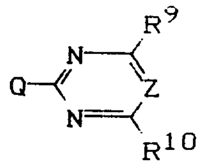

(III)

in welcher

| | |
|---|---|
| $R^9$, $R^{10}$ und Z | die in Anspruch 1 angegebenen Bedeutungen haben und |
| Q | für eine nucleofuge Abgangsgruppe steht, |

gegebenenfalls in Gegenwart eines Säureakzeptors und gegebenenfalls in Gegenwart eines Verdünnungsmittels umsetzt und die freien Säuren ($R^1$ = OH) gegebenenfalls - nach üblichen Methoden - in ihre Salze oder andere funktionelle Derivate überführt.

3. Herbizide Mittel, gekennzeichnet durch einen Gehalt an mindestens einer Verbindung der Formel (I) gemäß Anspruch 1.

4. Verwendung von Verbindungen der allgemeinen Formel (I) gemaß Anspruch 1 zur Bekämpfung von unerwünschtem Pflanzenwachstum.

5. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

6. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

7. Bicyclo[3.1.0]-hexane der Formel (II),

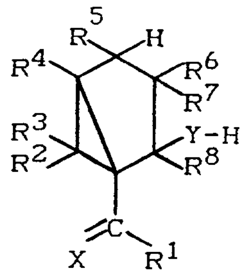

(II)

in welcher
$R^1$, $R^2$, $R^3$, $R^4$, $R^5$, $R^6$, $R^7$, $R^8$, X und Y die in AnSpruch 1 angegebene Bedeutungen haben.

**8.** Bicyclische Ketone der Formel (IVa),

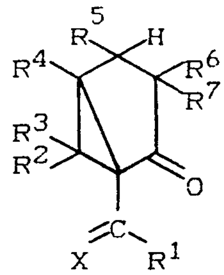

(IVa)

worin

- $R^1$ für Wasserstoff, Hydroxy, Amino oder für einen jeweils gegebenenfalls substituierten Rest aus der Reihe Alkyl, Alkoxy, Alkenyloxy, Alkinyloxy, Aryloxy, Heteroaryloxy, Alkylthio, Arylthio, Heteroarylthio, Alkylamino, Dialkylamino, Alkylsulfonylamino, Arylsulfonylamino oder Hydrazino steht,
- $R^2$ für Wasserstoff, Halogen, Alkyl, Alkoxy, Aryl oder Alkoxycarbonyl steht,
- $R^3$ für Wasserstoff oder Alkyl oder - wenn $R^2$ Wasserstoff, Alkyl oder Halogen bedeutet - auch für Halogen steht,
- $R^4$ für Wasserstoff, Halogen, Alkyl, Aryl oder Alkoxycarbonylalkyl steht,
- $R^5$ für Wasserstoff, Alkyl oder Aryl steht,
- $R^6$ für Wasserstoff, Hydroxy, Amino, Halogen, Cyano, Alkyl, Alkoxy, Aryl oder Alkoxycarbonyl steht,
- $R^7$ für Wasserstoff oder Alkyl oder - wenn $R^6$ Wasserstoff, Alkyl oder Halogen bedeutet - auch für Halogen steht,
- $R^8$ für Wasserstoff steht,
- X für Sauerstoff steht und
- Y für Sauerstoff steht,

- ausgenommen die Verbindungen:
2-Keto-6,6-dimethyl-bicyclo[3.1.0]hexan-1-carbonsäure-methylester,2-Keto-6-methyl-bicyclo[3.1.0]-hexan-1-carbonsäure-ethylester und 2-Keto-6-n-propyl-bicyclo[3.1.0]-hexan-1-carbonsäure-ethylester.

**9.** Verfahren zur Herstellung von bicyclischen Ketonen der Formel (IV),

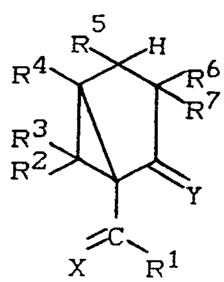

(IV)

worin

- $R^1$ für Alkoxy steht,
- $R^2$ für Wasserstoff, Halogen, Alkyl, Alkoxy, Aryl oder Alkoxycarbonyl steht,
- $R^3$ für Wasserstoff oder Alkyl oder - wenn $R^2$ Wasserstoff, Alkyl oder Halogen bedeutet - auch für Halogen steht,
- $R^4$ für Halogen, Alkyl, Aryl oder Alkoxycarbonylalkyl steht,
- $R^5$ für Wasserstoff, Alkyl oder Aryl steht,
- $R^6$ für Wasserstoff, Hydroxy, Amino, Halogen, Cyano, Alkyl, Alkoxy, Aryl oder Alkoxycarbonyl steht,
- $R^7$ für Wasserstoff oder Alkyl oder - wenn $R^6$ Wasserstoff, Alkyl oder Halogen bedeutet - auch für Halogen steht und

X für Sauerstoff steht,
dadurch gekennzeichnet, daß man - nach folgendem Reaktionsschema -

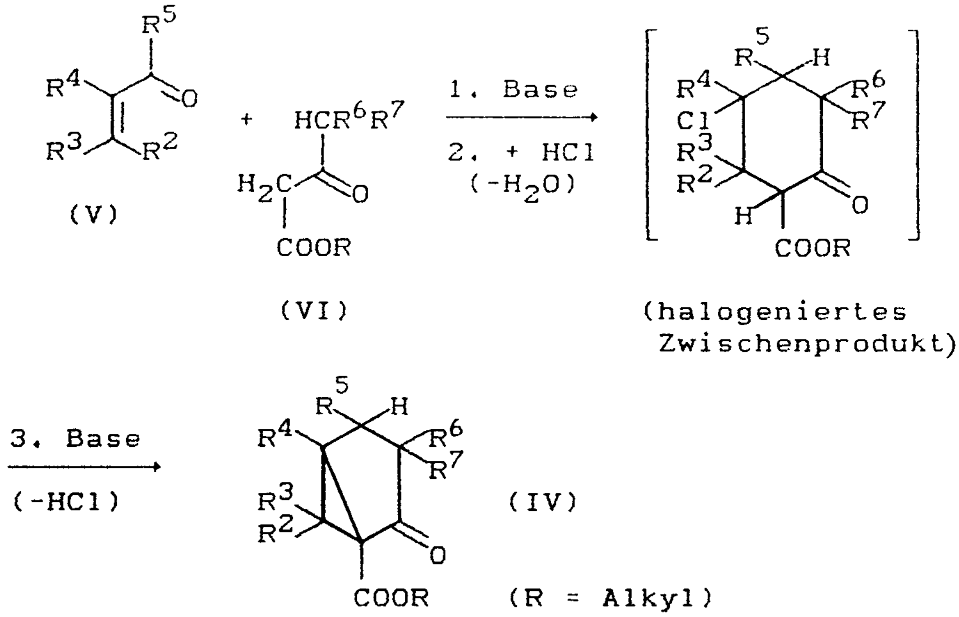


(V) (VI) (halogeniertes Zwischenprodukt) (IV) (R = Alkyl)

(α) eine α.β-ungesättigte Carbonylverbindung der Formel (V) mit einem β-Keto-ester der Formel (VI), wobei in den Formeln (V) und (VI)
$R^1$ bis $R^7$ und X die bei Formel (IV) genannten Bedeutungen haben,
in etwa äquimolaren Mengen zunächst in Gegenwart einer starken Base und in Gegenwart eines polaren Lösungsmittels bei Temperaturen zwischen -50 °C und +100 °C, vorzugsweise zwischen -40 °C und +80 °C, besonders bevorzugt zwischen 0 °C und +40 °C, umsetzt,
(β) das Reaktionsprodukt anschließend - ohne Zwischenisolierung - bei etwa 0 °C mit wasserfreiem Chlorwasserstoff (durch Sättigen der Lösung mit HCl-Gas) umsetzt und
(γ) schließlich das hierbei gebildete halogenierte Zwischenprodukt, z.B. durch Destillation, isoliert und dieses erneut mit einer Base bei Temperaturen zwischen 0 °C und 150 °C, vorzugsweise zwischen 20 °C und 120 °C, besonders bevorzugt zwischen 50 °C und 100 °C, zur Umsetzung bringt, wobei unter Halogenwasserstoff-Eliminierung die Bildung des Dreiringes und damit des Bicyclo[3.1.0]hexangerüstes erfolgt.